# EUROPEAN PATENT SPECIFICATION

(11) **EP 1 454 992 B1**
(45) Date of publication and mention of the grant of the patent: **31.05.2006**
(21) Application number: 03005186.6
(22) Date of filing: 07.03.2003
(51) Int. Cl.: C12Q 1/48, C07K 14/72, G01N 33/74, A61K 31/00

(54) **Anaplastic lymphoma kinase assay, reagents and compositions thereof**
Anaplastisches Lymphoma Kinase Testverfahren, Reagenzien und Kompositionen davon
Essai de kinase lymphome anaplastique, ses réactifs et compositions

(43) Date of publication of application: 08.09.2004
(73) Proprietor: ISTITUTO NAZIONALE PER LO STUDIO E LA CURA DEI TUMORI, I-20133 Milano (IT)
(72) Inventor: Pinna, Lorenzo A., 20133 Milano (IT); Donella-Deana, Arianna, 20133 Milano (IT); Marin, Oriano, 20133 Milano (IT); Mologni, Luca, 20133 Milano (IT); Gunby, Rosalind, 20133 Milano (IT); Gambacorti Passerini, Carlo, 20133 Milano (IT); Scapozza, Leonardo, 20133 Milano (IT)
(74) Representative: Banfi, Paolo

(56) References cited:
- EP-A- 0 575 955
- WO-A-95/02187
- WO-A-95/14930
- WO-A-98/49317
- US-A- 5 770 421
- DATABASE WPI Section Ch, Week 199325 Derwent Publications Ltd., London, GB; Class A96, AN 1993-201126 XP002249142 & JP 05 126833 A (TOSOH CORP), 21 May 1993 (1993-05-21)
- SADICK M D ET AL: "Kinase receptor activation (KIRA): A rapid and accurate alternative to end-point bioassays" JOURNAL OF PHARMACEUTICAL AND BIOMEDICAL ANALYSIS 1999 NETHERLANDS, vol. 19, no. 6, 1999, pages 883-891, XP002249139 ISSN: 0731-7085
- TURTURRO FRANCESCO ET AL: "Model of inhibition of the NPM-ALK kinase activity by herbimycin A." CLINICAL CANCER RESEARCH: AN OFFICIAL JOURNAL OF THE AMERICAN ASSOCIATION FOR CANCER RESEARCH. UNITED STATES JAN 2002, vol. 8, no. 1, January 2002 (2002-01), pages 240-245, XP002249140 ISSN: 1078-0432
- MORRIS STEPHAN W ET AL: "ALK, the chromosome 2 gene locus altered by the t(2;5) in non-Hodgkin's lymphoma, encodes a novel neural receptor tyrosine kinase that is highly related to leukocyte tyrosine kinase (LTK)." ONCOGENE, vol. 14, no. 18, 1997, pages 2175-2188, XP002249141 ISSN: 0950-9232
- SAUSVILLE, EDWARD A. ET AL: "Phase I trial of 72-hour continuous infusion UCN-01 in patients with refractory neoplasms" JOURNAL OF CLINICAL ONCOLOGY (2001), 19(8), 2319-2333, XP008024621
- HALLAHAN, DENNIS E. ET AL: "Inhibition of protein kinases sensitizes human tumor cells to ionizing radiation" RADIATION RESEARCH (1992), 129(3), 345-50, XP008024620
- VIRCHIS, ANDRES ET AL: "A novel treatment approach for low grade lymphoproliferative disorders using PKC412 (CGP41251), an inhibitor of protein kinase C" HEMATOLOGY JOURNAL (2002), 3(3), 131-136, XP008024623
- HARVEY, SARAH ET AL: "Interactions between 2-fluoroadenine 9-.beta.-D-arabinofuranoside and the kinase inhibitor UCN-01 in human leukemia and lymphoma cells" CLINICAL CANCER RESEARCH (2001), 7(2), 320-330, XP002261605
- SENDEROWICZ, A. M.: "Development of cyclin-dependent kinase modulators as novel therapeutic approaches for hematological malignancies" LEUKEMIA (2001), 15(1), 1-9, XP002261606
- GROSIOS, KONSTANTINA: "Midostaurin, Novartis AG" CURRENT OPINION IN ONCOLOGIC, ENDOCRINE & METABOLIC INVESTIGATIONAL DRUGS (2000), 2(1), 92-103, XP008024624
- GROSIOS, KONSTANTINA: "UCN-01 Kyowa Hakko Kogyo Co" CURRENT OPINION IN INVESTIGATIONAL DRUGS (PHARMAPRESS LTD.) (2001), 2(2), 287-297, XP008024634

## Description

The present invention provides an assay for measuring the kinase activity of the Anaplastic lymphoma kinase (ALK). More specifically the invention is directed to methods, reagents and compositions for detecting the tyrosine-phosphorylating activity of ALK. The assay is particularly useful for in vitro screening and identification of potential ALK inhibitors.

### BACKGROUND OF THE INVENTION

Anaplastic lymphoma kinase (ALK) is a receptor tyrosine kinase, believed to play an important role in the development and function of the nervous system. ALK is normally expressed in the central nervous system, with peak expression during the neonatal period. However, due to chromosomal translocations, ALK is also aberrantly expressed and activated in some cancers in the form of oncogenic fusion proteins. ALK fusion proteins are responsible for approximately 5 - 10% of all non-Hodgkin's lymphomas. The annual incidence of ALK positive lymphomas is about 100 000 worldwide, with 2000 - 3000 new cases occurring in EU countries. ALK is an excellent candidate for therapeutic intervention, as it plays an essential role in oncogenicity and its normal expression is mostly restricted to the central nervous system. Hence, a specific ALK inhibitor could be an efficient treatment for ALK positive lymphomas with few associated clinical side effects. For the identification of potential ALK inhibitors it is highly desirable to develop a suitable assay for assessing directly the inhibitory effect of compounds on enzyme activity.

### DESCRIPTION OF THE INVENTION

According to the invention, an *in vitro* kinase assay specific for ALK and useful for screening compounds that modulate ALK activity is provided. This assay is based on the use of a peptide substrate which is readily phosphorylated by ALK and on the subsequent detection of the phosphorylated product thus obtained. In a preferred embodiment of the invention the assay is an ELISA wherein the phosphorylated product is detected by immunochemical reactions.

In order to generate a selective ALK substrate, peptides reproducing the sequence of ALK activation loop (aa 1274-1294: ALK_HUMAN, Q9UM73, swiss-PROT) were synthesized and tested. The peptides ARDIYRASFFRKGGCAMLPVK (SEQ ID N. 1) and ARDIYRASYYRKGGCAMLPVK (SEQ ID N.2) were particularly effective as ALK substrates showing a phosphorylation degree higher than that of polyGlu/Tyr, a random polymer which is known to be a good substrate for most tyrosine kinases. The first object of the invention is therefore a peptide having an amino acid sequence selected from SEQ ID N. 1 and SEQ ID N. 2.

A further object of the invention is a method for detecting ALK tyrosine kinase activity, which essentially comprises the steps of:
i) incubating the ALK protein or a functional derivative thereof with a peptide selected from SEQ ID N. 1 and 2 in conditions suitable for phosphorylation of the peptide;
ii) detecting the phosphorylated peptide thus formed.

As used herein, "ALK functional derivative" means any modified form of ALK protein, for example a truncated or conjugated form or a fragment thereof, which maintains the catalytic activity of unmodified ALK. The functional derivative should preferably contain the entire catalytic domain of ALK spanning residues 1116-1392 of ALK sequence (Q9UM73). The portion of ALK protein stretching from residue Leu¹⁰⁷³ to Ala¹⁴⁵⁹ is preferably used. When produced by recombinant gene technology using the baculovirus-based expression system, this ALK fragment shows a correct folding (confirmed by CD spectra) and an effective catalytic activity.

In addition, a preparation containing a constitutively active form of ALK may be used instead of the purified protein or its functional derivative. Such a preparation is preferably a cell lysate, which could be used to a) assess for ALK activity in whole cells expressing ALK or ALK fusion proteins and b) assess for the effect of potential inhibitors on ALK within a cell, by treating ALK-expressing cells with different compounds and then assaying cell lysate for ALK kinase activity.

To detect the phosphorylated peptide in step ii), the phosphorylation reaction of step i) can be performed in the presence of a radioactive reagent, such as [γ³²P]ATP or [γ³³P]ATP, whereby a radioactive product is formed which is easily detected by radiometric techniques. Alternatively, an immunoreaction can be carried out, which comprises the formation of an immune complex between the phosphorylated peptide and an anti-phosphotyrosine antibody. This anti-phosphotyrosine antibody can be radioactively labelled or conjugated to a reporter enzyme, such as horse-radish peroxidase, beta-galactosidase, alkaline phosphatase or glucose oxidase, thereby allowing the direct detection of the antibody and thus of the phosphorylated peptide by measuring the specific radioactivity or enzymatic activity. As a further alternative, the anti-phosphotyrosine antibody may be detected indirectly by a second antibody recognizing the anti-phosphotyrosine antibody and carrying a radioactive label or a reporter enzyme, or by an enzyme conjugated streptavidin which recognises a biotin label on the anti-phosphotyrosine antibody.

In a preferred embodiment the invention provides a method for detecting ALK tyrosine kinase activity which comprises the steps of:
a) adhering a peptide of SEQ ID N. 1 or 2 to a solid phase;
b) incubating the solid phase with an ALK fragment extending from Leu¹⁰⁷³ to Ala¹⁴⁵⁹ in suitable conditions for tyrosine phosphorylation;
c) washing the solid phase;
d) incubating the solid phase with an anti-phosphotyrosine antibody (primary antibody) in conditions suitable for antigen-antibody binding;
e) washing the solid phase;
f) incubating the solid phase with an enzyme-conjugated antibody (secondary antibody) recognizing the anti-phosphotyrosine primary antibody in conditions suitable for binding of primary antibody to secondary antibody, so that a ternary immune complex is formed;
g) washing the solid phase;
h) measuring the enzymatic activity of the immune complex wherein the measured activity is proportional to the amount of tyrosine-phosphorylation.

This assay, which is properly an ELISA-based kinase assay, utilizes enzyme-antibody conjugates. The conjugated enzyme cleaves a substrate to generate a colored reaction product that can be detected spectrophotometrically by measuring the absorbance of the colored solution, which is proportional to the amount of phosphotyrosines.

Solid phases or supports which can be used according to the invention comprise plastic material (reaction plates, wells, vials), polystyrene, latice and magnetic beads, colloidal metal particles, glass and/or silica surfaces and others.

The ELISA-based ALK assay is preferably used for the screening of compounds that modulate the tyrosine-phosphorylating activity of ALK, in particular for screening ALK inhibitors.

In a preferred embodiment the invention is therefore directed to a method for the identification of compounds that modulate ALK tyrosine-kinase activity, wherein the ALK assay described above is carried out in the presence of a candidate compound or of a compound known to stimulate or inhibit ALK tyrosine kinase activity (control). Specifically, the method for identifying compounds that modulate ALK tyrosine-kinase activity comprises the steps of
i) incubating ALK protein or a functional derivative thereof with a peptide selected from SEQ ID N. 1 or 2, preferably SEQ ID N. 1, in the presence of a candidate compound, in conditions suitable for phosphorylation of the peptide;
ii) quantifying the phosphorylated peptide thus formed;
Compounds that inhibit ALK activity are selected as potential therapeutic agents for use in the treatment of ALK-related tumors, such as anaplastic large cell lymphomas and non-Hodgkin lymphomas. The ALK-modulating activity of a candidate compound can be compared to that of a reference compound (control), which is assayed under the same conditions as the candidate compound. Staurosporine (Meggio F. et al, Eyr. J. Biochem. (1995) 234, 317-322), which proved effective as an ALK inhibitor at 0.2 - 0.3 µM concentration, can be used as a positive control when screening other compounds for ALK inhibitory activity.
Studies of molecular modeling gave important indications as to the pattern of substitutions required to improve staurosporine affinity and specificity towards ALK protein. The most effective structures are represented in the following general formula (I): 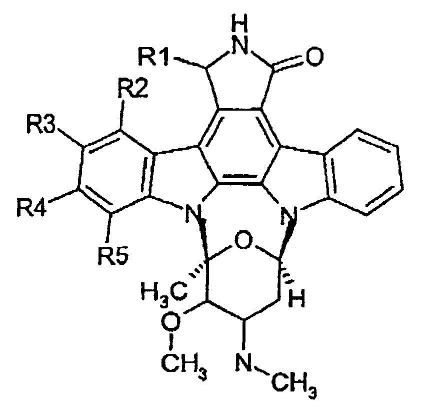 Wherein R1 and R2, independently of one another, are selected from halogen, preferably chlorine, phenyl or C1-C3 alkyl optionally substituted with one or more halogens; R3 is hydroxyl; R4 is hydroxyl or hydroxymethyl; R5 is C1-C3 alkyl, optionally halo-substituted, or benzyl.
The compounds of formula (I) antagonize the binding of ATP to the tyrosine kinase domain of ALK within oncogenic fusion proteins such as NPM-ALK, ATIC-ALK, CTLC-ALK, TFG-ALK or other sporadic fusions with tropomyosins.
In a different embodiment of the invention, compounds (I) are used in an ALK-assay for the screening of ALK-inhibitors, as herein disclosed. High-throughput screenings can also be implemented using the ALK assay of the invention.

According to a further aspect, the invention is directed to a kit for carrying out the ALK assay described above. The kit consists of a packaged combination of reagents in predetermined ratios. Typically the kit will contain a peptide of SEQ ID N: 1 or 2, optionally immobilized on a solid phase, the anti-phosphotyrosine antibody and, if necessary, an antibody labelled with an enzymatic or radioactive label. In addition the kit may contain reagents for colorimetric or radiometric reactions, buffers, controls such as staurosporine or a derivative thereof, diluents, detergents, stabilizers and any other component useful for setting up and carrying out the assay. The relative amounts of the various reagents may be varied to optimize the sensitivity of the assay. Particularly the reagents may be provided in solid or liquid preparations, such as solution, suspension, dispersion, dry powders, lyophilized preparation. The kit components are supplied in single or separate containers. The kit may also include instructions for carrying out the assay.

### DESCRIPTION OF THE FIGURES

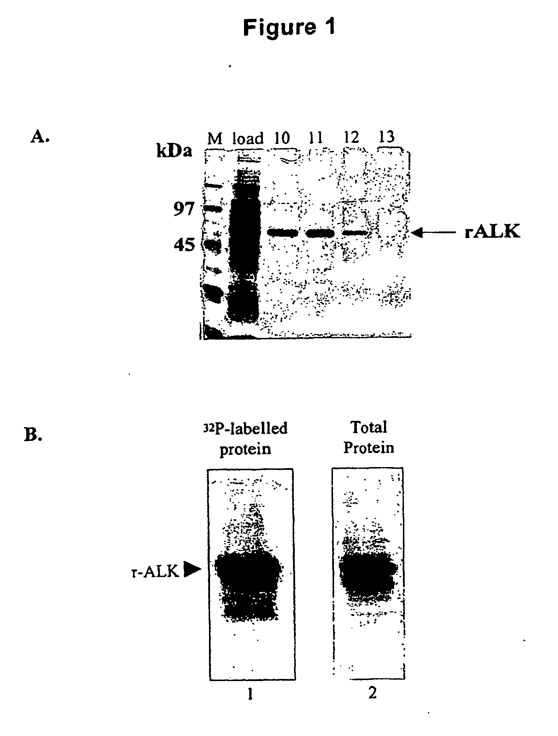

**Figure 1:** purification and activity of recombinant His-tagged ALK
   A) Silver stained 10% SDS-PAGE gel showing marker (M), dialysed and pooled DEAE column fractions loaded onto the HiTrap column (load), HiTrap column fractions (numbers indicate fraction number). B) Radioactive autophosphorylation assay of purified rALK. Lane 1: autoradiograph of 32P-labelled rALK. Lane 2: silver staining of the same sample as in Lane 1.
**Figure 2:** kinetics for ALK kinase with peptides
**Figure 3:** Detection of rALK activity using the ELISA based kinase assay
   A) Phosphorylation of peptide SEQ ID N.2 by purified rALK. An ELISA kinase reaction was performed with or without 0.5 µg purified rALK, 15 µg peptide SEQ. ID N.2 or no peptide, at 30°C for 30 mins. The graph shows absorbance at 450 nm. B) The kinetics of ALK peptide substrate phosphorylation. An ELISA kinase reaction was performed using 206 M peptide SEQ. ID N.2 or 42 µM peptide SEQ. ID N.1 with 0.1 µg of purified rALK. The reaction was stopped by adding EDTA after 0, 0.5, 2, 5, 10 and 15 mins.

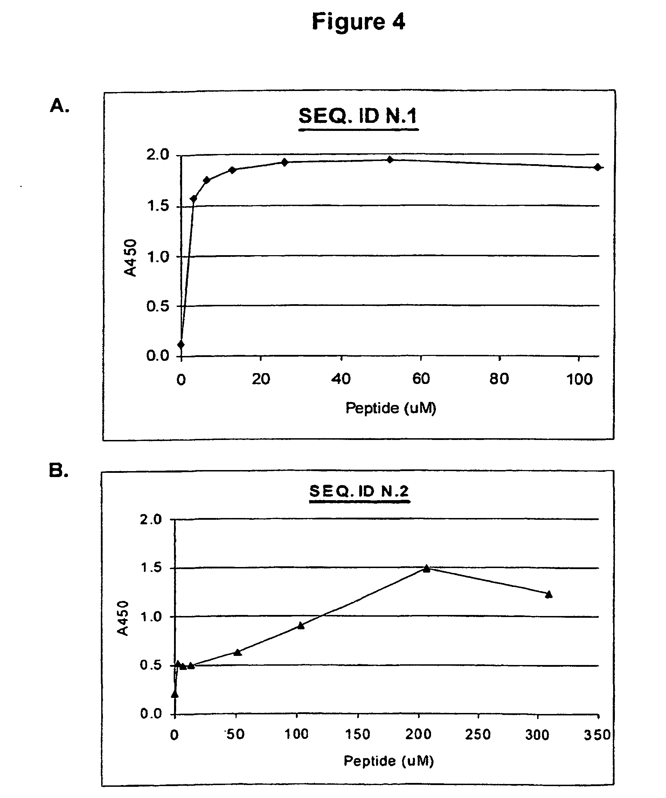

**Figure 4:** The effect of peptide substrate concentration on level of phosphorylation
   The ELISA assay was performed in the presence of 0.2 µg purified rALK and 0 - 105 µM peptide SEQ ID N.1 (A) or 0 - 315 µM peptide SEQ ID N.2 (B), at 30°C for 15 mins.

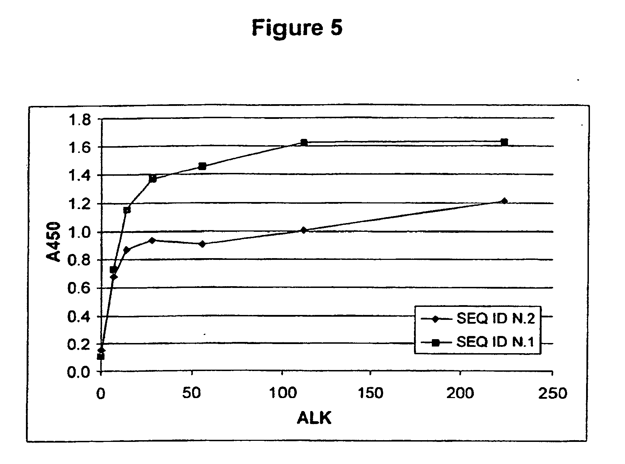

**Figure 5:** The effect of rALK concentration on substrate phosphorylation
   An ELISA assay was performed using 206 µM peptide SEQ ID N.2 or 42 µM peptide SEQ ID N.1 and rALK ranging from 0 - 225 ng, at 30°C for 15 mins.

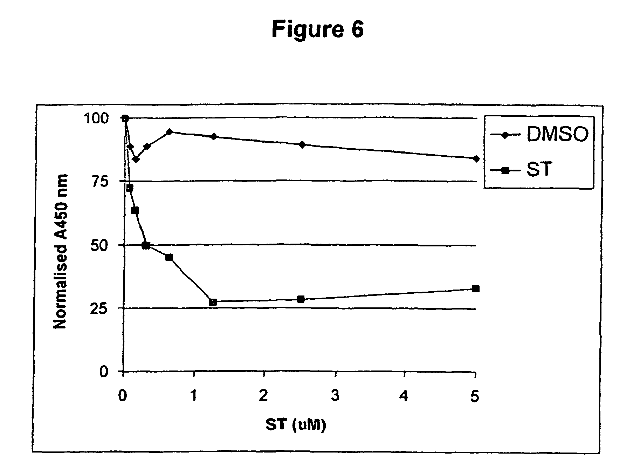

**Figure 6:** Inhibition of rALK activity by Staurosporine
   An ELISA assay was performed using 42 µM SEQ ID N.1, 20 ng rALK, at 30°C for 15 mins in the presence of 0 - 5 µM staurosporine or an equivalent volume of solvent, DMSO. Graph shows A450 normalised to the untreated control.

### EXPERIMENTAL RESULTS

### Peptide synthesis

Peptides were synthesised by automatic solid phase synthesis utilising 9-fluorenilmethoxycarbonyl (Fmoc) chemistry with an Applied Biosystems Model 431A synthesiser on 4-hydroxymethyl-copolystirene-1% divinylbenzene-resin (0.95 mmol/g, 0.05 mmol).

Fmoc amino acids (0.5 mmol) were activated by 2-(1H-benzotriazol-1-yl)-1,1,3,3-tetramethyluronium hexafluorophosphate (HBTU) (1 eq) and 1-hydroxybenzotriazole (HOBt) (1 eq) in the presence of DIPEA (2 eq).

Peptidyl-resins (100 mg) were cleaved and deprotected in a mixture containing 5 ml of trifluoroacetic acid, 0.375 gr of phenol, 0.125 µl of 1,2-ethanedithiol, 0.250 µl of thioanisole, and 0.25 µl of water for 2 hours. The reaction mixture was filtered onto a tube containing ethyl ether cooled at 0°C. Precipitated peptides were separated by centrifugation and washed with fresh ether.

Crude peptides (50-100 mg in 10 ml of water) were pumped onto a preparative RP-column (prepNova-Pak HR C18, 6 µm, 25×10 mm, Waters) and eluted with a linear gradient of 10% to 45% acetonitrile at 12 ml/min.

The purity of peptides was > 90% as judged by analytical RP-HPLC on a 5 µm, C18 Symmetry300 column, 4.6x250 mm (Waters) using a linear gradient of acetonitrile in 0.1% trifluoroacetic acid at 1 ml/min. Peptide molecular weights were confirmed by mass spectroscopy using a matrix-assisted laser-desorption ionisation time-of-flight (MALDI-Tof) spectrometer (Maldi-1; Kratos-Schimadzu, Mancester, UK).

### Production and purification of recombinant ALK

A portion of ALK, stretching from amino acids Leu¹⁰⁷³ to Ala¹⁴⁵⁹, has been cloned into the baculovirus transfer vector, pBlueBacHis2C (Invitrogen). Using this vector and the MaxBac® 2.0 baculovirus expression system (Invitrogen) we have produced recombinant baculovirus and expressed recombinant ALK (rALK) protein in Sf9 (*Spodoptera frugiperda*) insect cells. This protein has a theoretical molecular weight of 49 kDa and contains the predicted catalytic domain of ALK (Ile¹¹¹⁶ to Val¹³⁹²) fused to a 6-histidine-tag (His-tag). The autophosphorylation activity of His-tagged rALK, which is indicative of correct folding, has been verified by anti-phosphotyrosine immunoblotting of whole cell lysates and an *in vitro* radioactive kinase assay.

To produce protein for purification, Sf9 cells were infected with recombinant virus at a multiplicity of infection (MOI) of 5. Cultures were incubated for 3 days at 27°C and then harvested by centrifuging at 400 g for 10 minutes at 4°C. Cell pellets were stored at -80°C until use. To lyse the cells, cell pellets were resuspended in hypotonic buffer (Buffer A) containing 50 mM Tris-HCl pH 8, 20 mM NaCl and protease inhibitors (Pepstatin, Benzamidine, Leupeptin and Aprotinin). After 30 minutes incubation on ice, the cell suspension was centrifuged at 1500 g for 15 minutes at 4°C and the supernatant was filtered through a 0.45 µm filter. The filtrate was then loaded onto an 80 ml anion exchange Fast Flow DEAE-sepharose (Sigma) column in the presence of Buffer A at a flow of 1.5 ml/min using the AKTA FPLC system (Amersham-Pharmacia Biotech). Bound proteins were eluted in gradient of NaCl ranging from 20 to 200 mM. Fractions were analysed for the presence of ALK protein by immunoblotting. Positive fractions were pooled and dialysed for 3 h at 4°C in 1 litre of native binding buffer (20 mM sodium phosphate pH 7.8, 500 mM NaCl and PI), changing the buffer every hour. Dialysed fractions were then loaded onto a HiTrap^{™}-nickel affinity chromatography column (Amersham-Pharmacia Biotech), previously equilibrated with native binding buffer supplemented with 50 mM imidazole and 20 mM β-mercaptoethanol. A flow of 0.5 - 1 ml/min was applied to the column. After washing the column with native binding buffer bound proteins were eluted using a linear gradient of imidazole ranging from 50 to 200 mM. Fractions were then analysed for the presence and purity of rALK by SDS-PAGE and silver staining. Figure 1A shows an example purification of rALK.

Finally, positive fractions were dialysed in 50 mM Tris pH 7.4, 100 mM NaCl, 10% glycerol, 20 mM β-mercaptoethanol and protease inhibitors, and stored at -80°C until use. To verify that purified rALK was active we have performed a radioactive autophosphorylation assay. A reaction mix containing 25 µl of a rALK positive fraction, 6 µM cold ATP, 1.2 mM DTT, 10 µCi [γ-³²P]ATP, 25 mM Hepes pH 7.5, 10 mM MgCl₂ and 10 mM MnCl₂ was incubated at 30°C for 30 minutes. The reaction was stopped by adding Laemmli buffer and heating at 95°C for 5 minutes. The samples were resolved on a 10% SDS-PAGE gel and transferred to an Immobilon^{™}-P membrane. Radioactively labelled proteins were visualized by autoradiography as shown in Figure 1B.

### Peptide phosphorylation (radioactive assay)

Peptides and the random polymer polyGlu/Tyr (1:4) were phosphorylated in 30 µl of a medium containing 50 mM Tris/HCl, pH 7.5, 5 mM MnCl₂, 10 µM Na-vanadate, 30 µM [γ³²P]ATP or [γ³³P]ATP (specific activity 1000 cpm/pmol) and 10 units of rALK [one unit was defined as the amount of enzyme transferring 1 pmol phosphate per min to polyGluTyr (0.1 mg/ml)]. The reactions were terminated after 10 min of incubation at 30°C by spotting 25 µl of the mixture onto P81 phosphocellulose papers, which were processed as described elsewhere (1). Kinetic constants were determined by GraphPad Prism software fitting the data directly to the Michaelis-Menten equation using nonlinear regression.

### Results

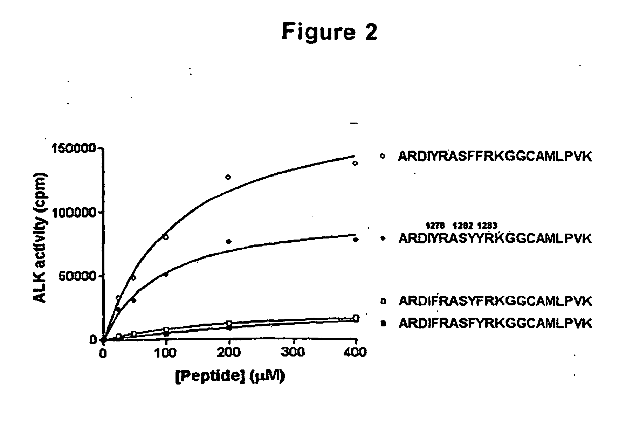

The peptide derived from ALK reference sequence, containing Tyr-1278 was a good substrate of rALK, whereas peptides containing either Tyr-1282 or Tyr-1283 were slightly affected by the enzyme (see Fig. 2). The Tyr-1278-derivative was phosphorylated with an efficiency even higher than that displayed by the peptide bearing three Tyr residues (Table I). To confirm that Tyr-1278-derivative is an optimal peptide substrate for ALK catalytic domain, we compared its phosphorylation degree with that displayed by polyGlu/Tyr (1:4), a random polymer that is a very good substrate for most tyrosine kinases. Table II shows that Tyr-1278-derivative is a better ALK substrate than polyGlu/Tyr.

**TABLE I Kinetic constants for rALK with synthetic peptides.**

| PEPTIDE | Vₘₐₓ | Kₘ | Efficiency |
|---|---|---|---|
| | (pmol/min) | (µM) | (Vₘₐₓ/Kₘ) |
| ARDIYRASYYRKGGCAMLPVK | 99.5 | 90.5 | 1.1 |
| ARDIYRASFFRKGGCAMLPVK | 186.3 | 109.4 | 1.7 |

**TABLE II Phosphorylation rates of model substrates by ALK catalytic domain. Poly(Glu/Tyr) and peptide concentrations were 0.1 mg/ml and 400 µM, respectively. Enzyme concentration was 10 units. Reported values represent the means for three separate experiments. S.E.M. values were always less than 14%.**

| SUBSTRATE | Phosphorylation degree |
|---|---|
| | (pmol/min) |
| Poly(Glu/Tyr) | 10.0 |
| ARDIYRASFFRKGGCAMLPVK | 30.3 |

### ELISA-based in vitro kinase assay

A Nunc Immuno 96 well plate was incubated overnight at 37°C with coating solution (125 µl/ well) containing ALK peptide substrate (SEQ ID N. 1 or SEQ ID N. 2) at various concentrations in PBS. The wells were then washed with 200 µl of wash buffer (PBS-Tween 0.05 %) and left to dry for at least 2 hours at 37°C. The kinase reaction was performed by incubating kinase buffer (25 mM Hepes pH 7.5, 5 mM MnCl₂, 5 mM MgCl₂), 0.3 mM ATP and purified rALK at various concentrations in a total volume of 100 µl/well at 30°C for 15 minutes. The reaction mix was then removed and wells were washed 5 times with 200 µl of wash buffer. Phosphorylated peptide was detected using 100 µl/well of a mouse monoclonal anti-phosphotyrosine antibody (clone 4G10 UpstateBiotech Ltd) diluted 1:500 in PBS + 4% BSA. After 30 minutes incubation at room temperature the antibody was removed and wells were washed as described above. 100 µl of a secondary antibody (anti-mouse IgG, Horseradish Peroxidase linked whole antibody, from sheep, Amersham Pharmacia Biotech) diluted 1:1000 in PBS + 4% BSA was added to each well and the plate was incubated again for 30 minutes at room temperature before washing as above. The plate was developed using 100 µl/well TMB Substrate Solution (Endogen) and the reaction was stopped by adding an equal volume of H₂SO₄ 0.18 M. Finally, the absorbance was read at 450 or 490 nm using an Ultrospec® 300 spectrophotometer (Amersham-Pharmacia Biotech).

### Results

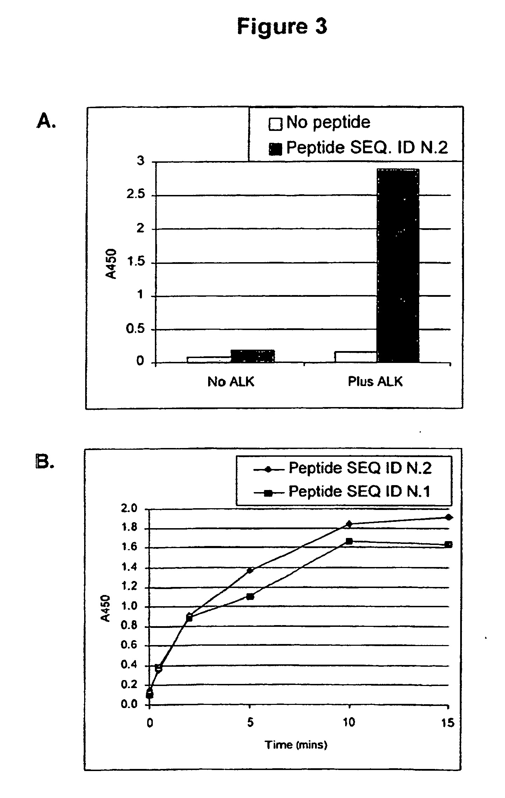

We have shown that the ELISA-based kinase assay can be used to detect phosphorylated ALK peptide substrates (SEQ ID N.1 and N.2) and hence the activity of purified rALK. In initial experiments we performed the assay in the presence and absence of both purified rALK and the peptide substrate SEQ ID N.2, in order to determine if a specific increase in absorbance at 450 nm (A450) could be observed (Figure 3A). Indeed in the presence of both rALK and substrate a dramatic increase in A450 was observed. In contrast, in the absence of either peptide or rALK or both, A450 was low, indicating a low level of background absorbance. To determine the kinetics of substrate phosphorylation we measured A450 after various incubation times (Figure 3B). For both peptides the kinetics of the reaction was similar with maximum phosphorylation achieved after 10 minutes.

To compare the efficiency of the two peptides in the ELISA kinase assay and to determine the optimal concentration of peptide to use we performed standard curves for both peptides. Results show that increasing the amount of peptide substrate increased A450 and hence rALK kinase activity. For the peptide substrate SEQ ID N.2, maximum A450 and hence phosphorylation was observed at approximately 200 µM, compared with only 25 µM for peptide substrate SEQ ID N.1 (Figures 4A and B). This indicates that peptide substrate SEQ ID N.1 is more efficient as a substrate for rALK.

To observe inhibition of kinase activity it is essential that we use an appropriate concentration of enzyme, i.e. in the linear range of a standard curve. An ELISA was performed using 206 µM SEQ ID N.2 or 42 µM SEQ ID N.1 and rALK ranging from 0 - 220 ng. The linear range of the curve for both substrates was approximately between 0 - 15 ng of rALK, after which the curves reached plateau (Figure 5).

The effect of the inhibitor staurosporine on rALK activity was assessed using 42 µM peptide substrate SEQ ID N.2. A solvent control containing DMSO at corresponding concentrations was also performed. Staurosporine markedly inhibited rALK activity reaching maximum inhibition at 1 µM and with an estimated IC₅₀= 300 nM (Figure 6). The solvent (DMSO) alone had no substantial effect on rALK activity.

### REFERENCES

[1] Glass, D.B., Masaracchia, R.A., Feramisco, J.R. and Kemp, D.E. (1978) Anal. Biochem. 87, 566-575.
[2] Till, J.H., Ablooglu, A.J., Frankel, M., Bishop, S.M., Kohanski, R.A. and Hubbard S.R. (2001) J. Biol. Chem. 276, 10049-10055.

## Claims

1. A method for detecting ALK tyrosine kinase activity, which comprises the following steps:
i) incubating the ALK protein or a functional derivative thereof with a peptide substrate selected from SEQ ID N. 1 or 2 in conditions suitable for phosphorylation of the peptide;
ii) detecting the phosphorylated peptide.

2. A method according to claim 1, wherein the peptide has sequence SEQ ID N. 1.

3. A method according to claim 1, wherein purified ALK protein or an ALK-containing preparation is used.

4. A method according to claim 3, wherein said preparation is a cell lysate.

5. A method according to claim 1, wherein said functional derivative contains the entire catalytic domain of ALK spanning residues 1116-1392 of ALK sequence.

6. A method according to claim 5, wherein said functional derivative is a fragment of ALK protein extending from residue Leu¹⁰⁷³ to Ala¹⁴⁵⁹.

7. A method according to claim 6, which comprises the steps of:
a) adhering a peptide of SEQ ID N. 1 or 2 to a solid phase;
b) incubating the solid phase with said ALK fragment in conditions suitable for tyrosine phosphorylation;
c) washing the solid phase;
d) incubating the solid phase with an anti-phosphotyrosine antibody (primary antibody) in conditions suitable for antigen-antibody binding;
e) washing the solid phase;
f) incubating the solid phase with an enzyme-conjugated antibody (secondary antibody) recognizing the primary antibody in conditions suitable for the binding of primary and secondary antibodies, so that a ternary immune complex is formed;
g) washing the solid phase;
h) measuring the enzymatic activity of the immune complex wherein the measured activity is proportional to the amount of tyrosine-phosphorylation.

8. A method according to claim 7, wherein the enzyme conjugated to the antibody is Horse-Radish peroxidase.

9. A method according to claim 7, wherein the enzymatic activity is detected by colorimetric reaction.

10. A method according to any previous claims, for the identification of compounds that modulate ALK tyrosine-kinase activity.

11. A method according to claim 10, which comprises the steps of
i) incubating ALK protein or a functional derivative thereof with a peptide selected from SEQ ID N. 1 or 2 in the presence of a candidate compound (a) in conditions suitable for peptide phosphorylation;
ii) detecting the phosphorylated peptide thus formed;

12. A method according to claims 10-11, wherein the ALK-modulating activity of the candidate compound is compared to that of a reference compound which is assayed under the same conditions as the candidate compound.

13. A method according to claim 12, wherein the reference compound is staurosporine.

14. A method according to claim 12, wherein the reference compound is a staurosporine derivative of general formula (I): 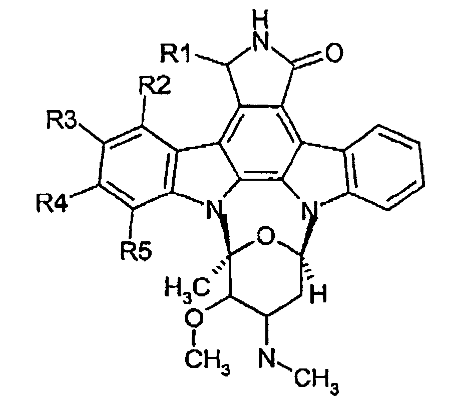 Wherein R1 and R2, independently of one another, are selected from halogen, preferably chlorine, phenyl or C1-C3 alkyl optionally substituted with one or more halogens; R3 is hydroxyl; R4 is hydroxyl or hydroxymethyl; R5 is C1-C3 alkyl, optionally halo-substituted, or benzyl.

15. A peptide useful as ALK substrate selected from SEQ ID N. 1 or 2.

16. A peptide acording to claim 15, which is SEQ ID N. 1.

17. The use of a peptide according to claim 15 or 16 for the determination of ALK tyrosine-kinase activity.

18. A kit for detecting ALK tyrosine-kinase activity according to claims 1-14, which comprises a peptide of SEQ ID N: 1 or 2 and an anti phosphotyrosine antibody.

19. A kit according to claim 18, containing an additional component selected from reagents for colorimetric reactions, buffers, diluents, detergents, stabilizers, staurosporine or a derivative thereof as per claim 14.

## Patentansprüche

1. Verfahren zum Nachweis einer ALK-Tyrosinkinaseaktivität, welches die folgenden Schritte umfasst:
i) Inkubation des ALK-Proteins oder eines funktionellen Derivats davon mit einem Peptidsubstrat, das aus SEQ ID Nr. 1 oder 2 ausgewählt ist, unter Bedingungen, die für die Phosphorylierung des Peptids geeignet sind;
ii) Nachweis des phosphorylierten Peptids.

2. Verfahren nach Anspruch 1, worin das Peptid die Sequenz SEQ ID Nr. 1 hat.

3. Verfahren nach Anspruch 1, wobei ein gereinigtes ALK-Protein oder eine ALK-enthaltende Zubereitung verwendet wird.

4. Verfahren nach Anspruch 3, wobei die Zubereitung ein Zelllysat ist.

5. Verfahren nach Anspruch 1, wobei das funktionelle Derivat die gesamte katalytische Domäne von ALK enthält, die sich von den Resten 1116 bis 1392 der ALK-Sequenz erstreckt.

6. Verfahren nach Anspruch 5, wobei das funktionelle Derivat ein Fragment des ALK-Proteins ist, das sich vom Rest Leu¹⁰⁷³ bis Ala¹⁴⁵⁹ erstreckt.

7. Verfahren nach Anspruch 6, welches die folgenden Schritte umfasst:
a) Binden des Peptids mit SEQ ID Nr. 1 oder 2 an eine feste Phase;
b) Inkubation der festen Phase mit dem ALK-Fragment unter Bedingungen, die für die Tyrosinphosphorylierung geeignet sind;
c) Waschen der festen Phase;
d) Inkubation der festen Phase mit einem Anti-Phosphotyrosin-Antikörper (primärer Antikörper) unter Bedingungen, die für eine Antigen-Antikörper-Bindung geeignet sind;
e) Waschen der festen Phase;
f) Inkubation der festen Phase mit einem enzymkonjugierten Antikörper (sekundärer Antikörper), der den primären Antikörper erkennt, unter Bedingungen, die für die Bindung des primären und des sekundären Antikörpers geeignet sind, so dass ein dreifacher Immunkomplex gebildet wird;
g) Waschen der festen Phase;
h) Messen der enzymatischen Aktivität des Immunkomplexes, wobei die gemessene Aktivität proportional zu dem Betrag der Tyrosinphosphorylierung ist.

8. Verfahren nach Anspruch 7, wobei das Enzym, das mit dem Antikörper konjugiert ist, eine Meerrettichperoxidase ist.

9. Verfahren nach Anspruch 7, wobei die enzymatische Aktivität durch eine kotorimetrische Reaktion nachgewiesen wird.

10. Verfahren nach einem der vorangegangenen Ansprüche für die Identifikation von Verbindungen, welche die ALK-Tyrosinkinaseaktivität modulieren.

11. Verfahren nach Anspruch 10, das die folgenden Schritte umfasst:
i) Inkubation des ALK-Proteins oder eines funktionellen Derivats davon mit einem Peptid, das aus SEQ ID Nr. 1 oder 2 ausgewählt ist, in Gegenwart einer Kandidatenverbindung (a) unter Bedingungen, die für die Peptidphosphorylierung geeignet sind;
iii) Nachweis des so gebildeten, phosphorylierten Peptids.

12. Verfahren nach den Ansprüchen 10-11, wobei die ALK-modulierende Aktivität der Kandidatenverbindung mit der einer Referenzverbindung verglichen wird, die unter den gleichen Bedingungen wie die Kandidatenverbindung getestet wird.

13. Verfahren nach Anspruch 12, wobei die Referenzverbindung Staurosporin ist.

14. Verfahren nach Anspruch 12, wobei die Referenzverbindung ein Staurosporin-Derviat der allgemeinen Formel (I) ist: 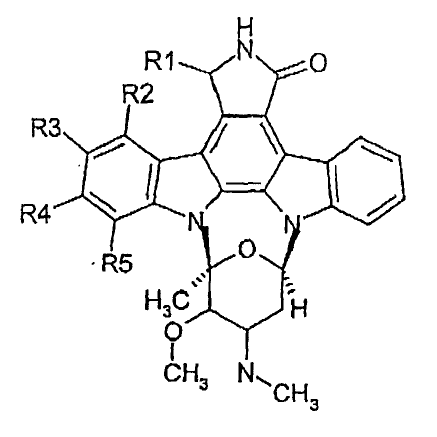 wobei R1 und R2 unabhängig voneinander aus Halogen, vorzugsweise Chlor, Phenyl oder C1-C3 Alkyl, das optinat mit einem oder mehreren Halogenen substituiert ist, ausgewählt werden; R3 Hydroxyl ist; R4 Hydroxyl oder Hydroxymethyl ist; R5 C1-C3 Alkyl, optional halogensubstituiert ist, oder Benzyl ist.

15. Peptid, das als ALK-Substrat nützlich ist und aus SEQ ID Nr. 1 oder 2 ausgewählt ist.

16. Peptid nach Anspruch 15, welches SEQ ID Nr. 1 ist.

17. Verwendung des Peptids nach Anspruch 15 oder 16 für die Bestimmung der ALK-Tyrosinkinaseaktivität.

18. Kit zum Nachweis der ALK-Tyrosinkinaseaktivität nach einem der Ansprüche 1 bis 14, das ein Peptid mit SEQ ID Nr. 1 oder 2 und einen Anti-Phosphotyrosin-Antikörper umfasst.

19. Kit nach Anspruch 18, der eine zusätzliche Komponente enthält, die ausgewählt ist aus Reagenzien für kotorimetrische Reaktionen, Puffer, Verdünnungsmittel, Reinigungsmittel, Stabilisatoren, Staurosporin oder einem Derivat davon gemäß Anspruch 14.

## Revendications

1. Procédé de détection de l'activité tyrosine kinase d'ALK, qui comprend les étapes suivantes :
i) incubation de la protéine ALK ou d'un dérivé fonctionnel de celle-ci avec un substrat peptidique choisi parmi SEQ ID N. 1 ou 2 dans des conditions appropriées pour la phosphorylation du peptide ;
ii) détection du peptide phosphorylé.

2. Procédé selon la revendication 1, dans lequel le peptide a la séquence SEQ ID N. 1.

3. Procédé selon la revendication 1, dans lequel on utilise la protéine ALK purifiée ou une préparation contenant de l'ALK.

4. Procédé selon la revendication 3, dans lequel ladite préparation est un lysat de cellules.

5. Procédé selon la revendication 1, dans lequel ledit dérivé fonctionnel contient le domaine catalytique entier des résidus recouvrant ALK 1116-1392 de la séquence d'ALK.

6. Procédé selon la revendication 5, dans lequel ledit dérivé fonctionnel est un fragment de protéine ALK s'étendant du résidu Leu¹⁰⁷³ à Ala¹⁴⁵⁹.

7. Procédé selon la revendication 6, qui comprend les étapes de :
a) adhésion d'un peptide de SEQ ID N. 1 ou 2 à une phase solide ;
b) incubation de la phase solide avec ledit fragment d'ALK dans des conditions appropriées pour la phosphorylation de tyrosines ;
c) lavage de la phase solide ;
d) incubation de la phase solide avec un anticorps anti-phosphotyrosine (anticorps primaire) dans des conditions appropriées pour la liaison antigène-anticorps ;
e) lavage de la phase solide ;
f) incubation de la phase solide avec un anticorps conjugué à une enzyme (anticorps secondaire) reconnaissant l'anticorps primaire dans des conditions appropriées pour la liaison des anticorps primaire et secondaire, de sorte qu'un complexe immun ternaire est formé ;
g) lavage de la phase solide ;
h) mesure de l'activité enzymatique du complexe immun où l'activité mesurée est proportionnelle à la quantité de phosphorylation de tyrosines.

8. Procédé selon la revendication 7, dans lequel l'enzyme conjuguée à l'anticorps est la peroxydase de raifort.

9. Procédé selon la revendication 7, dans lequel l'activité enzymatique est détectée par réaction colorimétrique.

10. Procédé selon l'une quelconque des revendications précédentes, pour l'identification de composés qui modulent l'activité tyrosine kinase d'ALK.

11. Procédé selon la revendication 10, qui comprend les étapes de :
i) incubation de la protéine ALK ou d'un dérivé fonctionnel de celle-ci avec un peptide choisi parmi SEQ ID N. 1 ou 2 en présence d'un composé candidat (a) dans des conditions appropriées pour la phosphorylation du peptide ;
ii) détection du peptide phosphorylé ainsi formé.

12. Procédé selon les revendications 10-11, dans lequel l'activité modulant ALK du composé candidat est comparée à celle d'un composé de référence qui est testé dans les mêmes conditions que le composé candidat.

13. Procédé selon la revendication 12, dans lequel le composé de référence est la staurosporine.

14. Procédé selon la revendication 12, dans lequel le composé de référence est un dérivé de staurosporine de formule générale (I) : 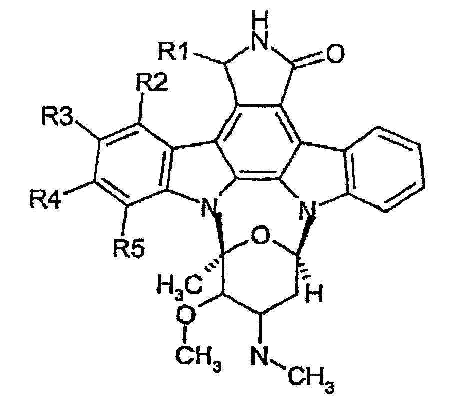 dans laquelle R1 et R2, indépendamment l'un de l'autre, sont choisis parmi un halogène, de préférence le chlore, un groupe phényle ou un groupe (C1-C3)-alkyle éventuellement substitué par un ou plusieurs halogènes ; R3 est un groupe hydroxyle ; R4 est un groupe hydroxyle ou hydroxyméthyle ; R5 est un groupe (C1-C3)alkyle, éventuellement substitué par un halogène, ou un groupe benzyle.

15. Peptide utile comme substrat d'ALK choisi parmi SEQ ID N. 1 ou 2.

16. Peptide selon la revendication 15, qui est SEQ ID N. 1.

17. Utilisation d'un peptide selon la revendication 15 ou 16 pour la détermination de l'activité tyrosine kinase d'ALK.

18. Trousse pour la détection de l'activité tyrosine kinase d'ALK selon les revendications 1-14, qui comprend un peptide de SEQ ID N: 1 ou 2 et un anticorps anti-phosphotyrosine.

19. Trousse selon la revendication 18, contenant un composant supplémentaire choisi parmi les réactifs pour réactions colorimétriques, les tampons, les diluants, les détergents, les stabilisants, la staurosporine ou un de ses dérivés conformément à la revendication 14.


### SEQUENCE LISTING

<110> ISTITUTO NAZIONALE PER LO STUDIO E LA CURA DEI TUMORI
<120> ANAPLASTIC LYMPHOMA KINASE ASSAY, REAGENTS AND COMPOSITIONS THEREOF
<130> 1206EUR
<160> 2
<170> PatentIn version 3.1
<210> 1
   <211> 21
   <212> PRT
   <213> Unknown
<220>
   <223> synthetic peptide
   <400> 1
   Ala Arg Asp Ile Tyr Arg Ala Ser Phe Phe Arg Lys Gly Gly Cys Ala
   1               5                   10                  15
   Met Leu Pro Val Lys
               20
<210> 2
   <211> 21
   <222> PRT
   <213> Unknown
<220>
   <223> synthetic peptide
   <400> 2
   Ala Arg Asp Ile Tyr Arg Ala Ser Tyr Tyr Arg Lys Gly Gly Cys Ala
   1               5                   10                  15
   Met Leu Pro Val Lys
               20